# EUROPEAN PATENT APPLICATION

(11) **EP 4 238 551 A1**
(43) Date of publication of application: **06.09.2023**
(21) Application number: 21886454.4
(22) Date of filing: 03.03.2021
(51) Int. Cl.: A61K 8/88, A61K 8/34, A61Q 19/00

(54) **PRESERVATIVE COMPRISING POLYLYSINE AND CAPRYLYL GLYCERYL ETHER FOR EXTERNAL-USE SKIN FORMULATIONS AND COSMETICS COMPOSITION COMPRISING SAME**

(30) Priority: 30.10.2020 KR 20200143891
(71) Applicant: Activon Co., Ltd., Cheongju-si, Chungcheongbuk-do (KR)
(72) Inventor: KIM, Myo Deok, Cheongju-si, Chungcheongbuk-do 28113 (KR); LEE, Jun Hak, Cheongju-si, Chungcheongbuk-do 28115 (KR); CHO, Myung Chan, Seoul 06310 (KR); JUNG, Sung Won, Yongin-si, Gyeonggi-do 16909 (KR); PARK, Byung Gyu, Suwon-si, Gyeonggi-do 16668 (KR); CHO, Youn Ki, Yongin-si, Gyeonggi-do 17005 (KR)
(74) Representative: Weickmann & Weickmann PartmbB
(86) International application number: PCT/KR2021/002617
(87) International publication number: WO 2022/092453

(57) **Abstract**

The present application relates to a preservative comprising polylysine and caprylyl glyceryl ether and the use thereof, and provides a preservative for a skin external preparation, which exhibits excellent antimicrobial or preservative effects against bacteria and fungi, and a cosmetics composition comprising the preservative.

## Description

### Technical Field

The present invention relates to a preservative for a skin external preparation comprising polylysine and caprylyl glyceryl ether, and a cosmetic composition containing the same. This patent application claims priority to Korean Patent Application No. 10-2020-0143891 filed with the Korean Intellectual Property Office on October 30, 2020, the disclosure of which is incorporated herein by reference.

### Background Art

Cosmetic products cannot avoid microbial contamination during the production or use thereof, and thus preservatives capable of improving the preservative activity of the products are essentially required for producing the cosmetic products. Examples of widely used preservatives include paraben preservatives, imidazolidinyl urea, phenoxyethanol, and the like. The above-mentioned preservatives have the advantage of having excellent preservative activity due to their high antimicrobial activity, but are also widely known to have the disadvantage of causing toxicity, skin irritation, allergy, and the like. There is a need to develop a preservative that causes no skin irritation and has high preservative activity and safety.

As part of research to overcome this disadvantage, the development of preservatives using caprylyl glyceryl ether known to have antimicrobial activity have been continued, and Korean Patent Application Publication No. 2019-0035047 discloses the use of caprylyl glyceryl ether as a preservative. However, when a large amount of caprylyl glyceryl ether is applied to a cosmetic product in order to increase preservative effect, there is still a problem in that caprylyl glyceryl ether may cause stability degradation such as precipitation.

### DISCLOSURE

### Technical Problem

The present inventors have developed a preservative for a skin external preparation comprising polylysine and caprylyl glyceryl ether, and have found that the preservative has excellent antimicrobial activity, preservative activity, safety and stability, thereby completing the present invention.

An object of the present invention is to provide a preservative for a skin external preparation comprising polylysine and caprylyl glyceryl ether.

Another object of the present invention is to provide the preservative for a skin external preparation further comprising a glycol compound.

Still another object of the present invention is to provide a cosmetic composition containing the preservative for a skin external preparation.

However, objects to be achieved by the present invention are not limited to the above-mentioned objects, and other problems not mentioned herein will be clearly understood by those skilled in the art from the following description.

### Technical Solution

One aspect provides a preservative for a skin external preparation comprising polylysine and caprylyl glyceryl ether.

As used herein, the term "polylysine" refers to a polymer of lysine. The polylysine may be referred to as poly(Lys), (Lys)n, or the like. Specifically, the polylysine may be polyepsilon-lysine.

As used herein, the term "polyepsilon-lysine" may be named epsilon-polylysine, poly epsilon L-lysine, poly(L-lysine) macromolecule, etc., and refers to a polyamide comprising the amino acid lysine (L-Lysine) connected between carboxyl and epsilon amino groups by an amide bond.

The preservative for a skin external preparation may comprise about 0.01 to 10 wt% of the polylysine. Specifically, the preservative for a skin external preparation may comprise about 0.01 to 5 wt%, about 0.01 to 1 wt%, about 0.01 to 0.1 wt%, about 0.1 to 10 wt%, about 0.1 to 5 wt%, about 0.1 to 3 wt%, about 0.1 to 1 wt%, about 1 to 10 wt%, about 1 to 5 wt%, about 1 to 3 wt%, about 3 to 10 wt%, about 3 to 5 wt%, or about 5 to 10 wt% of the polylysine. Preferably, the preservative for a skin external preparation may comprise about 0.1 to 5 wt% or about 0.1 to 1 wt% of the polylysine. According to one embodiment, if the preservative for a skin external preparation comprises the polylysine in an amount of less than about 0.01 wt%, the antimicrobial or preservative effect thereof may decrease, and if the preservative for a skin external preparation comprises the polylysine in an amount of more than about 10 wt%, it may cause stability degradation such as solidification, precipitation, reduced solubility, reduced emulsifying capacity, or reduced solubilizing capacity.

The preservative for a skin external preparation may comprise about 10 to 35 wt% of the caprylyl glyceryl ether. Specifically, the preservative for a skin external preparation may comprise about 10 to 30 wt%, about 10 to 25 wt%, about 10 to 20 wt%, about 10 to 15 wt%, about 15 to 35 wt%, about 15 to 30 wt%, about 15 to 25 wt%, about 15 to 20 wt%, about 20 to 35 wt%, about 20 to 30 wt%, about 20 to 25 wt%, about 25 to 35 wt%, about 25 to 30 wt%, or about 30 to 35 wt% of the caprylyl glyceryl ether. Preferably, the preservative for a skin external preparation may comprise about 10 to 30 wt%, about 15 to 30 wt%, about 15 to 25 wt%, about 20 to 30 wt%, or about 25 to 30 wt% of the caprylyl glyceryl ether. According to one embodiment, if the preservative for a skin external preparation comprises the caprylyl glyceryl ether in an amount of less than about 10 wt%, the antimicrobial or preservative effect thereof may decrease, and if the preservative for a skin external preparation comprises the caprylyl glyceryl ether in an amount of more than about 35 wt%, it may cause stability degradation such as solidification, precipitation, reduced solubility, reduced emulsifying capacity, or reduced solubilizing capacity.

The preservative for a skin external preparation may comprise about 0.1 to 5 wt% or about 0.1 to 1 wt% of polylysine, and about 10 to 30 wt%, about 15 to 30 wt%, about 15 to 25 wt%, about 20 to 30 wt%, or about 25 to 30 wt% of caprylyl glyceryl ether. Alternatively, the preservative for a skin external preparation may comprise 0.1 to 5 parts by weight or 0.1 to 1 part by weight of polylysine, and 10 to 30 parts by weight, 15 to 30 parts by weight, 15 to 25 parts by weight, 20 to 30 parts by weight, 25 to 30 parts by weight, or 25 parts by weight of caprylyl glyceryl ether. According to one embodiment, if the amounts or proportions of polylysine and caprylyl glyceryl ether in the preservative for a skin external preparation are out of the above ranges of wt% or parts by weight, the antimicrobial or preservative effect of the preservative for a skin external preparation may significantly decrease, or solidification, precipitation, reduced solubility, reduced emulsifying capacity or reduced solubilizing capacity may occur, and thus the stability of the preservative for a skin external preparation may significantly decrease.

The weight ratio between the polylysine and the caprylyl glyceryl ether in the preservative for a skin external preparation may be 0.1 to 5: 10 to 30. Specifically, the weight ratio between the polylysine and the caprylyl glyceryl ether in the preservative for a skin external preparation may be 0.1 to 5: 10 to 25, 0.1 to 5: 10 to 20, 0.1 to 5: 10 to 15, 0.1 to 5: 15 to 30, 0.1 to 5: 15 to 25, 0.1 to 5: 15 to 20, 0.1 to 5: 20 to 30, 0.1 to 5: 20 to 25, 0.1 to 5: 25 to 30, 0.1 to 5: 25, 0.1 to 3: 10 to 30, 0.1 to 3: 10 to 25, 0.1 to 3: 10 to 20, 0.1 to 3: 10 to 15, 0.1 to 3: 15 to 30, 0.1 to 3: 15 to 25, 0.1 to 3: 15 to 20, 0.1 to 3: 20 to 30, 0.1 to 3: 20 to 25, 0.1 to 3: 25 to 30, 0.1 to 3: 25, 0.1 to 1: 10 to 30, 0.1 to 1: 10 to 25, 0.1 to 1: 10 to 20, 0.1 to 1: 10 to 15, 0.1 to 1: 15 to 30, 0.1 to 1: 15 to 25, 0.1 to 1: 15 to 20, 0.1 to 1: 20 to 30, 0.1 to 1: 20 to 25, 0.1 to 1: 25 to 30, or 0.1 to 1: 25. According to one embodiment, if the weight ratio between the polylysine and the caprylyl glyceryl ether in the preservative for a skin external preparation is out of the ratio of 0.1 to 5: 10 to 30, the antimicrobial or preservative effect of the preservative for a skin external preparation may significantly decrease, or solidification, precipitation, reduced solubility, reduced emulsifying capacity or reduced solubilizing capacity may occur, and thus the stability of the preservative for a skin external preparation may significantly decrease.

The preservative for a skin external preparation may further comprise a glycol compound.

The glycol compound may be at least one selected from the group consisting of ethylene glycol, diethylene glycol, propylene glycol, dipropylene glycol, butylene glycol, pentylene glycol, hexylene glycol, ethoxydiglycol, and an alkanediol having 3 to 12 carbon atoms, without being limited thereto. In addition, the alkanediol having 3 to 12 carbon atoms may be octanediol, decanediol, propanediol, butanediol, pentanediol, hexanediol, heptanediol, undecanediol, dodecanediol, or a combination thereof, without being limited thereto. Preferably, the glycol compound may be at least one selected from the group consisting of propylene glycol, butylene glycol, pentylene glycol, propanediol, butanediol, and pentanediol.

The preservative for a skin external preparation may comprise about 60 to 80 wt% of the glycol compound. Specifically, the preservative for a skin external preparation may comprise about 60 to 78 wt%, about 60 to 75 wt%, about 60 to 73 wt%, about 60 to 70 wt%, about 60 to 68 wt%, about 60 to 65 wt%, about 60 to 63 wt%, about 63 to 80 wt%, about 63 to 78 wt%, about 63 to 75 wt%, about 63 to 73 wt%, about 63 to 70 wt%, about 63 to 68 wt%, about 63 to 65 wt%, about 65 to 80 wt%, about 65 to 78 wt%, about 65 to 75 wt%, about 65 to 73 wt%, about 65 to 70 wt%, about 65 to 68 wt%, about 68 to 80 wt%, about 68 to 78 wt%, about 68 to 75 wt%, about 68 to 73 wt%, about 68 to 70 wt%, about 70 to 80 wt%, about 70 to 78 wt%, about 70 to 75 wt%, about 70 to 73 wt%, about 73 to 80 wt%, about 73 to 78 wt%, about 73 to 75 wt%, about 75 to 80 wt%, about 75 to 78 wt%, or about 78 to 80 wt% of the glycol compound. Preferably, the preservative for a skin external preparation may comprise about 65 to 75 wt%, or about 70 to 75 wt% of the glycol compound. According to one embodiment, if the preservative for a skin external preparation may comprise the glycol compound in an amount of less than about 60 wt%, it may cause stability degradation such as solidification, precipitation, reduced solubility, reduced emulsifying capacity, or reduced solubilizing capacity, and if the preservative for a skin external preparation may comprise the glycol compound in an amount of more than about 80 wt%, the antimicrobial or preservative effect thereof may decrease.

The preservative for a skin external preparation may comprise about 0.1 to 5 wt% or about 0.1 to 1 wt% of polylysine, about 10 to 30 wt%, about 15 to 30 wt%, about 15 to 25 wt%, about 20 to 30 wt%, or about 25 to 30 wt% of caprylyl glyceryl ether, and about 65 to 75 wt% or about 70 to 75 wt% of the glycol compound. Alternatively, the preservative for a skin external preparation may comprise 0.1 to 5 parts by weight or 0.1 to 1 part by weight of polylysine, 10 to 30 parts by weight, 15 to 30 parts by weight, 15 to 25 parts by weight, 20 to 30 parts by weight, 25 to 30 parts by weight, or 25 parts by weight of caprylyl glyceryl ether, and 65 to 75 parts by weight or 70 to 75 parts by weight of the glycol compound. According to one embodiment, if the amounts or proportions of polylysine, caprylyl glyceryl ether and the glycol compound in the preservative for a skin external preparation are out of the above ranges of wt% or parts by weight, the antimicrobial or preservative effect of the preservative for a skin external preparation may significantly decrease, or solidification, precipitation, reduced solubility, reduced emulsifying capacity or reduced solubilizing capacity may occur, and thus the stability of the preservative for a skin external preparation may significantly decrease.

The weight ratio between the polylysine, the caprylyl glyceryl ether and the glycol compound in the preservative for a skin external preparation may be 0.1 to 5: 10 to 30: 65 to 75. Specifically, the weight ratio between the polylysine, the caprylyl glyceryl ether and the glycol compound in the preservative for a skin external preparation may be 0.1 to 5: 15 to 30: 65 to 75, 0.1 to 5: 15 to 25: 65 to 75, 0.1 to 5: 20 to 30: 65 to 75, 0.1 to 5: 25 to 30: 65 to 75, 0.1 to 5: 25: 65 to 75, 0.1 to 1: 10 to 30: 65 to 75, 0.1 to 1: 10 to 30: 70 to 75, 0.1 to 1: 15 to 30: 65 to 75, 0.1 to 1: 15 to 30: 70 to 75, 0.1 to 1: 15 to 25: 65 to 75, 0.1 to 1: 15 to 25: 70 to 75, 0.1 to 1: 20 to 30: 65 to 75, 0.1 to 1: 20 to 30: 70 to 75, 0.1 to 1: 25 to 30: 65 to 75, 0.1 to 1: 25 to 30: 70 to 75, 0.1 to 1: 25: 65 to 75, or 0.1 to 1: 25: 70 to 75. According to one embodiment, if the weight ratio between the polylysine, the caprylyl glyceryl ether and the glycol compound in the preservative for a skin external preparation is out of the range of 0.1 to 5: 10 to 30: 65 to 75, the antimicrobial or preservative effect of the preservative for a skin external preparation may significantly decrease, or solidification, precipitation, reduced solubility, reduced emulsifying capacity or reduced solubilizing capacity may occur, and thus the stability of the preservative for a skin external preparation may significantly decrease.

According to one embodiment, the preservative for a skin external preparation may exhibit an excellent antimicrobial or preservative effect and formulation stability. Specifically, when the preservative for a skin external preparation comprises the polylysine and the caprylyl glyceryl ether, it may exhibit a significantly increased antimicrobial or preservative effect compared to a preservative comprising only one of the polylysine and the caprylyl glyceryl ether, and at the same time, may exhibit optimized formulation stability that can stably maintain a liquid state under various temperature conditions, specifically, room temperature and refrigeration conditions, without occurrence of discoloration, off-odor, solidification, precipitation, reduced solubility, reduced emulsifying capacity, reduced solubilizing capacity, or the like. In addition, when the preservative for a skin external preparation further comprises the glycol compound in addition to the polylysine and the caprylyl glyceryl ether, it may exhibit an increased antimicrobial or preservative effect and formulation stability compared to the preservative not comprising the glycol compound. According to one embodiment, when the preservative for a skin external preparation comprises polyepsilon-lysine, caprylyl glyceryl ether, and propylene glycol, it may exhibit the best antimicrobial or preservative effect and formulation stability.

In addition, according to one embodiment, the preservative for a skin external preparation may exhibit excellent antimicrobial or preservative effects against various types of microorganisms (specifically, contaminating microorganisms). Specifically, the preservative for a skin external preparation is able to inhibit the growth of or kill Gram-positive bacteria, Gram-negative bacteria, and fungi (e.g., yeast, mold, etc.). Specifically, the preservative for a skin external preparation may exhibit excellent antimicrobial or preservative effects against Escherichia *coli, Pseudomonas aeruginosa, Staphylococcus aureus, Candida albicans,* and *Aspergillus brasiliensis,* without being limited thereto.

In addition, according to one embodiment, the preservative for a skin external skin preparation may exhibit excellent skin safety because it has significantly low skin irritation potential compared to preservatives (paraben preservatives, imidazolidinyl urea, phenoxyethanol, etc.), which are mainly used in existing cosmetic products, or the use of polylysine or caprylyl glyceryl ether alone.

As used herein, the term "skin external preparation" is meant to encompass all compositions for application to the skin, including, for example, various cosmetics such as basic cosmetics, makeup cosmetics, and hair cosmetics; and pharmaceutical compositions for topical administration, including pharmaceuticals and quasi-drugs such as ointments, creams, and lotions.

In the present specification, the term "preservative for a skin external preparation" may be used interchangeably with "antimicrobial agent", "antiseptic", "antimicrobial preservative", "preservative for antisepsis", "antimicrobial composition", "preservative composition", "antimicrobial or preservative composition", "antimicrobial cosmetic composition", "preservative cosmetic composition", "antimicrobial or preservative cosmetic composition" or the like.

As used herein, the term "antimicrobial" means resistance to all contaminating microorganisms, including bacteria, mold, and yeasts, the term "antimicrobial activity" or "antimicrobial effect" means a defense activity or defense effect against these contaminating microorganisms, the term "preservative activity" or "preservative effect" means a defense activity or defense effect against deterioration caused by contamination by microorganisms, and is meant to include antimicrobial activity or antimicrobial effect.

As used herein, the term "preservative" means a substance having a preservative or antioxidant activity that is capable of preventing deterioration of a product for a long period of time and maintaining the original state of the product, and the term "preservative activity" means a defense activity that is capable of preventing deterioration of a product for a long period of time and maintaining the original state of the product.

The preservative for a skin external preparation may further comprise additives such as purified water, a carrier, an emulsifier, a moisturizer, a skin conditioning agent, a surfactant, a chelating agent, an antioxidant, an antiseptic, a bactericide, a stabilizer, a preservative, a pH adjusting agent, a lubricant, a solubilizer, a solvent, etc. In addition, the preservative for a skin external preparation may further comprise a substance capable of supplementing essential nutrients to the skin or a substance for improving skin conditions, such as a functional substance having an effect such as wrinkle reduction or whitening. In addition, the preservative for a skin external preparation may further comprise adjuvants including, but not limited to, natural fragrance, cosmetic fragrance, or a plant extract.

Another aspect provides a cosmetic composition containing the preservative for a skin external preparation.

The cosmetic composition may contain the preservative for a skin external preparation in an amount of about 0.0001 to 50 wt% based on the total weight of the cosmetic composition. Specifically, the cosmetic composition may contain the preservative for a skin external preparation in an amount of about 0.0001 to 0.001 wt%, about 0.001 to 0.01 wt%, about 0.01 to 0.1 wt%, or about 0.1 to 1 wt%, about 1 to 10 wt%, about 10 to 20 wt%, about 20 to 30 wt%, about 30 to 40 wt%, or about 40 to 50 wt%, based on the total weight of the cosmetic composition. According to one embodiment, if the cosmetic composition contains the preservative for a skin external preparation in an amount of less than about 0.0001 wt% based on the total weight of the cosmetic composition, it may have a reduced antimicrobial or preservative effect, and if the cosmetic composition contains the preservative for a skin external preparation in an amount of more than about 50 wt% based on the total weight of the cosmetic composition, stability degradation such as solidification, precipitation, reduced solubility, reduced emulsifying capacity, or reduced solubilizing capacity may occur.

The cosmetic composition may further contain an additive selected from the group consisting of purified water, a carrier, an emulsifier, a moisturizer, a skin conditioning agent, a surfactant, a chelating agent, an antioxidant, an antiseptic, a bactericide, a stabilizer, a preservative, a pH adjusting agent, a lubricant, a solubilizer, a solvent, and combinations thereof, without being limited thereto. In addition, the cosmetic composition may further contain a substance capable of supplementing essential nutrients to the skin or a substance for improving skin conditions, such as a functional substance having an effect such as wrinkle reduction or whitening. In addition, the cosmetic composition may further contain adjuvants including, but not limited to, natural fragrance, cosmetic fragrance, or a plant extract.

The cosmetic composition may be prepared in any formulation that is commonly prepared in the art. Specifically, the formulation of the cosmetic composition may be a solution, emulsion, suspension, softening lotion, nourishing lotion, massage cream, nourishing cream, pack, gel, skin-adhesive type cosmetic, lipstick, powder, makeup base, foundation, shampoo, rinse, scalp cleanser, scalp lotion, scalp cream, scalp pack, scalp gel, scalp ointment, scalp gel, body cleanser, soap, toothpaste, mouth freshener, paste, lotion, ointment, gel, cream, patch, spray, or aerosol formulation, without being limited thereto.

The carrier that is contained in the cosmetic composition may be selectively used depending on the formulation of the cosmetic composition. For example, when the cosmetic composition is prepared in the form of ointment, paste, cream, or gel, wax, paraffin, starch, tragacanth, a cellulose derivative, polyethylene glycol, silicone, bentonite, silica, talc, zinc oxide, etc. may be used alone or in combination as a carrier component. When the cosmetic composition is prepared in the form of powder or spray, lactose, talc, silica, aluminum hydroxide, calcium silicate, polyamide powder, chlorofluorohydrocarbon, propane/butane, dimethyl ether, etc. may be used alone or in combination as a carrier component. When the cosmetic composition is prepared in the form of a solution or emulsion, water, ethanol, isopropanol, ethyl carbonate, ethyl acetate, benzyl alcohol, benzyl benzoate, propylene glycol, 1,3-butyl glycol oil, cottonseed oil, peanut oil, corn germ oil, olive oil, castor oil, sesame oil, glycerol aliphatic ester, polyethylene glycol, or sorbitan fatty acid ester, etc. may be used alone or in combination as a carrier component. When the cosmetic composition is prepared in the form of a suspension, water, ethanol or propylene glycol, ethoxylated isostearyl alcohol, polyoxyethylene sorbitol ester, polyoxyethylene sorbitan ester, microcrystalline cellulose, aluminum meta-hydroxide, bentonite, agar, tragacanth, etc. may be used alone or in combination as a carrier component. When the cosmetic composition is prepared in the form of soap, alkali metal salts of fatty acids, fatty acid hemiester salts, fatty acid protein hydrolyzates, isethionates, lanolin derivatives, aliphatic alcohols, vegetable oils, glycerol, sugars, etc. may be used alone or in combination as a carrier component.

Of the terms or elements mentioned above with respect to the cosmetic composition, those as mentioned in the description of the preservative for a skin external preparation are understood to be the same as those mentioned in the above description of the preservative for a skin external preparation.

Still another aspect provides a method for preparing a preservative for a skin external preparation, the method comprising mixing polylysine and caprylyl glyceryl ether, or mixing polylysine, caprylyl glyceryl ether and a glycol compound.

In the above method, the mixing weight ratio, wt%, or parts by weight of each component is as mentioned in the description of the preservative for a skin external preparation.

Yet another aspect provides a method of subjecting the skin external preparation or the cosmetic composition to antimicrobial, preservation, or antiseptic treatment using the preservative for a skin external preparation.

Of the terms or elements mentioned above with respect to the method, those as mentioned in the description of the preservative for a skin external preparation or the cosmetic composition are understood to be the same as those mentioned in the above description of the preservative for a skin external preparation or the cosmetic composition.

### Advantageous Effects

A preservative for a skin external preparation according to one aspect has remarkably excellent antimicrobial activity, preservative activity, and safety, and when it is applied to a cosmetic composition, it may be used as a skin-safe preservative, antiseptic, or antiseptic adjuvant.

In addition, the preservative for a skin external preparation has optimized formulation stability that can stably maintain a liquid state under various temperature conditions, specifically, room temperature and refrigeration conditions, without discoloration, off-odor, and precipitation, and thus it may be stably applied to a cosmetic composition by solving a stability degradation problem that may occur in a mixture of polylysine and caprylyl glyceryl ether.

### Mode for Invention

Hereinafter, the present invention will be described in more detail with reference to examples. However, these examples are for illustrating the present invention by way of example, and the scope of the present invention is not limited to these examples.

### 1. Preparation and evaluation of preservatives for skin external preparations comprising polylysine, caprylyl glyceryl ether, or combination thereof

### Example 1 to Example 5. Preparation of preservatives for skin external preparation

In these examples, preservatives for skin external preparations containing polylysine, caprylyl glyceryl ether, or a combination thereof were prepared. As the polylysine, polyepsilon-lysine was used.

Specifically, 5 types of preservatives for skin external preparations were prepared while changing the wt% of each ingredient mixed, and the results are shown in Table 1 below.

**[Table 1]**

| Ingredient name (unit: wt%) | **Example 1** | **Example 2** | **Example 3** | **Example 4** | **Example 5** |
|---|---|---|---|---|---|
| Caprylyl glyceryl ether | 10 | 25 | 50 | 75 | 90 |
| Polyepsilon-lysine | 45 | 37.5 | 25 | 12.5 | 5 |
| Purified water | Up to 100 | Up to 100 | Up to 100 | Up to 100 | Up to 100 |

### Experimental Example 1. Evaluation of the antimicrobial effect of preservatives for skin external preparations

In this experimental example, in order to evaluate the antimicrobial effect of the preservatives for skin external preparations, prepared in Examples 1 to 5, the minimum inhibitory concentrations (MICs) of the preservative of each Example against bacteria or fungi, including *Escherichia coli* (ATCC 8739), *Pseudomonas aeruginosa* (ATCC 9027), *Staphylococcus aureus* (ATCC 6538), *Candida albicans* (ATCC 10231), and *Aspergillus brasiliensis* (ATCC 16404), were measured.

Specifically, each of the preservatives for skin external preparations, prepared in Examples 1 to 5, was diluted to about 13% to about 25%, and each of the dilutions was further serially diluted 1: 1 (10 times). Thereby, samples of various concentrations (about 0.05% to about 10%) for each of the preservatives for skin external preparations, prepared in Examples 1 to 5, were prepared. Each of the obtained samples was added to tryptic soy broth (TSB) medium or potato dextrose agar (PDA) medium. In about 10 ml of the medium to which each sample was added, each type of bacteria or fungi described above was seeded so that the final concentration of the bacteria or fungi was about 10⁶ CFU/ml, and then the bacteria were cultured at about 32°C for about 24 hours to 48 hours, and fungi were cultured at about 25°C for about 24 hours to 48 hours. Thereafter, the minimum inhibitory concentration (MIC) of each sample capable of inhibiting the growth of the bacteria or fungi by about 95% or more was determined by observing the state of the bacterial or fungal culture medium. The growth of normal bacteria in a medium to which no sample was added was measured and used as a positive control, and the MIC for the growth of bacteria or fungi in a medium to which polyepsilon-lysine, caprylyl glyceryl ether, or propylene glycol was added alone was measured and used as a control.

As a result, as shown in Table 2, it was confirmed that the preservatives for skin external preparations, prepared in Examples 1 to 5 to comprise a combination of polyepsilon-lysine and caprylyl glyceryl ether, exhibited significantly better antimicrobial activity at significantly lower concentrations than when the bacteria or fungi were treated with polyepsilon-lysine, caprylyl glyceryl ether, or propylene glycol alone.

**[Table 2]**

| MIC (%, w/w) of sample | *E. coli* | *P. aeruginosa* | *S. aureus* | *C. albicans* | *A. brasiliensis* |
|---|---|---|---|---|---|
| Caprylyl glyceryl ether | 0.15 | 0.15 | 0.08 | <0.05 | <0.05 |
| Polyepsilon-lysine | <0.05 | <0.05 | 0.4 | <0.05 | 6.25 |
| Propylene glycol | >10 | >10 | >10 | >10 | >10 |
| Example 1 | 0.04 | 0.02 | 0.1 | <0.006 | >0.1 |
| Example 2 | 0.04 | 0.02 | 0.1 | <0.006 | 0.05 |
| Example 3 | 0.04 | 0.025 | 0.04 | 0.01 | 0.04 |
| Example 4 | 0.04 | 0.08 | 0.04 | 0.02 | 0.02 |
| Example 5 | 0.04 | 0.08 | 0.04 | 0.025 | 0.02 |

### 2. Preparation and evaluation of preservatives for skin external preparations comprising polylysine, caprylyl glyceryl ether, and glycol compound

### Example 6 to Example 50. Preparation of preservatives for skin external preparations having increased stability (determination of optimal composition ratio)

In the case of the preservatives for skin external preparations according to Examples 1 to 5, solidification or precipitation may occur, which may limit their application to cosmetic compositions. Therefore, in these Examples, preservatives for skin external preparations having further increased stability were prepared, thereby determining the optimal combination and composition ratio of components constituting the preservative for a skin external preparation.

Specifically, the stability of the preservative for a skin external preparation was optimized by additionally adding a glycol compound in addition to polylysine and caprylyl glyceryl ether and adjusting the composition ratio of each component. That is, preservatives for skin external preparations according to Examples 6 to 50, which comprise polylysine, caprylyl glyceryl ether, and a glycol compound at different composition ratios, were prepared and then stored under refrigeration conditions (about 2 to 8°C) for about 72 hours, and then the stability thereof was evaluated. Polyepsilon-lysine was used as the polylysine, and propylene glycol, butylene glycol, or pentylene glycol was used as the glycol compound.

As a result, as shown in Tables 3, 4, and 5 below, it was confirmed that the preservatives for skin external preparations according to Examples 6, 7, 9, 10, 12, 14, 15, 17, 21, 24, 27, 29, 30, 36, 39, 42, 44 and 45, which comprise polyepsilon-lysine, caprylyl glyceryl ether and the glycol compound at a weight ratio of 0.1 to 10: 5 to 35: 63 to 75, were stably maintained in a liquid state without occurrence of solidification or precipitation, indicating that they had significantly increased stability.

In particular, it was confirmed that, among the preservatives for skin external preparations according to Examples 6, 9, 12, 14, 15, 21, 24, 27, 29, 30, 36, 39, 42, 44 and 45, which comprise polyepsilon-lysine, caprylyl glyceryl ether and the glycol compound at a weight ratio of 0.1 to 5: 10 to 30: 65 to 75, the preservatives comprising propylene glycol, butylene glycol, or pentylene glycol as the glycol compound were stably maintained in a liquid state without occurrence of solidification or precipitation, indicating that they had significantly increased stability. In addition, it was confirmed that, when the composition ratio of polyepsilon-lysine, caprylyl glyceryl ether and the glycol compound in the preservative for a skin external preparation is out of the weight ratio of 0.1 to 5:10 to 30:65 to 75, solidification or precipitation occurred, indicating that the stability could be significantly reduced.

**[Table 3]**

| | | | | | | | | | | | | | | | |
|---|---|---|---|---|---|---|---|---|---|---|---|---|---|---|---|
| Ing red ien t nam e (wt %) | Example | | | | | | | | | | | | | | |
| | 6 | 7 | 8 | 9 | 10 | 11 | 12 | 13 | 14 | 15 | 16 | 17 | 18 | 19 | 20 |
| Cap ryl yl gly cer yl eth er | 25 | 38 | 45 | 25 | 35 | 45 | 25 | 35 | 15 | 25 | 35 | 5 | 15 | 5 | 15 |
| Pol yep sil on-lys ine | 0.1 | 0.1 | 0.1 | 1 | 1 | 1 | 3.5 | 3.5 | 5 | 5 | 5 | 10 | 10 | 15 | 10 |
| Pur ifi ed wat er | 0.1 | 0.1 | 0.1 | 1 | 1 | 1 | 3.5 | 3.5 | 5 | 5 | 5 | 10 | 10 | 15 | 10 |
| Pro pyl ene gly col | 74. 8 | 64 . 8 | 54 . 8 | 73 | 63 | 53 | 68 | 58 | 75 | 65 | 55 | 75 | 65 | 65 | 55 |
| Eva lua tie n | Sta ble | Sta ble | Sol idi fie d | Sta ble | Sta ble | Sol idi fie d | Sta ble | Sol idi fie d | Sta ble | Sta ble | Sol idi fie d | Sta ble | Pre cip ita ted /so lid ifi ed | Pre cip ita ted /so lid ifi ed | Pre cip ita ted /so lid ifi ed |

**[Table 4]**

| | | | | | | | | | | | | | | | |
|---|---|---|---|---|---|---|---|---|---|---|---|---|---|---|---|
| Ing red ien t nam e (wt %) | Example | | | | | | | | | | | | | | |
| | 21 | 22 | 23 | 24 | 25 | 26 | 27 | 28 | 29 | 30 | 31 | 32 | 33 | 34 | 35 |
| Cap ryl yl gly cer yl eth er | 25 | 35 | 45 | 25 | 35 | 45 | 25 | 35 | 15 | 25 | 35 | 5 | 15 | 5 | 15 |
| Pol yep sil on-lys ine | 0.1 | 0.1 | 0.1 | 1 | 1 | 1 | 3.5 | 3.5 | 5 | 5 | 5 | 10 | 10 | 15 | 15 |
| Pur ifi ed wat er | 0.1 | 0.1 | 0.1 | 1 | 1 | 1 | 3.5 | 3.5 | 5 | 5 | 5 | 10 | 10 | 15 | 15 |
| But yle ne gly col | 74. 8 | 64 . 8 | 54 . 8 | 7 3 | 63 | 53 | 68 | 58 | 75 | 65 | 55 | 75 | 65 | 65 | 55 |
| Eva luatie n | Sta ble | Sol idifie d | Sol idifie s | Sta ble | Sol idifie d | Sol idifie d | Sta ble | Sol idifie d | Sta ble | Sta ble | Sol idifie d | Pre cipita ted | Pre cipita ted /so lid ifi ed | Pre cipita ted /so lid ifi ed | Pre cipita ted /so lid ifi ed |

**[Table 5]**

| | | | | | | | | | | | | | | | |
|---|---|---|---|---|---|---|---|---|---|---|---|---|---|---|---|
| Ing red ien t nam e (wt %) | Example | | | | | | | | | | | | | | |
| | 36 | 37 | 38 | 39 | 40 | 41 | 42 | 43 | 44 | 45 | 46 | 47 | 48 | 49 | 50 |
| Cap ryl yl gly cer yl eth er | 25 | 35 | 45 | 25 | 35 | 45 | 25 | 35 | 15 | 25 | 35 | 5 | 15 | 5 | 15 |
| Pol yepsil on-lys ine | 0.1 | 0.1 | 0.1 | 1 | 1 | 1 | 3.5 | 3.5 | 5 | 5 | 5 | 10 | 10 | 15 | 15 |
| Pur ifi ed wat er | 0.1 | 0.1 | 0.1 | 1 | 1 | 1 | 3.5 | 3.5 | 5 | 5 | 5 | 10 | 10 | 15 | 15 |
| Pen tyl ene gly col | 74. 8 | 64. 8 | 54. 8 | 73 | 63 | 53 | 68 | 58 | 75 | 65 | 55 | 75 | 65 | 65 | 55 |
| Eva lua tie n | Sta ble | Sol idi fie d | Sol idi fie d | Sta ble | Sol idi fie d | Sol idi fie d | Sta ble | Sol idi fie d | Sta ble | Sta ble | Sol idi fie d | Pre cip ita ted | Pre cip ita ted /so lid ifi ed | Pre cip ita ted /so lid ifi ed | Pre cip ita ted /so lid ifi ed |

Through this Experimental Example, it was confirmed that the preservative for a skin external preparation comprising about 0.1 to 5 wt% of polylysine, about 10 to 30 wt% of caprylyl glyceryl ether and about 65 to 75 wt% of the glycol compound could overcome the solidification or precipitation problem, which may occur in a mixture of polylysine and caprylyl glyceryl ether, and could exhibit significantly increased stability. In addition, it was confirmed that, when the wt% of each of polylysine, caprylyl glyceryl ether and the glycol compound in the preservative for a skin external preparation was out of the above-described range of wt%, solidification or precipitation occurred, indicating that the stability of the preservative significantly decreased.

### Experimental Example 2. Evaluation of antimicrobial activity of preservatives for skin external preparations having increased stability

In this experimental example, in order to evaluate the antimicrobial effect of a preservative for a skin external application comprising polylysine, caprylyl glyceryl ether and a glycol compound at a weight ratio of 0.1 to 5: 10 to 30: 65 to 75, the minimum inhibitory concentrations (MICs) of the preservative of each Example against bacteria or fungi, including *Escherichia coli* (ATCC 8739), *Pseudomonas aeruginosa* (ATCC 9027), *Staphylococcus aureus* (ATCC 6538), *Candida albicans* (ATCC 10231), and *Aspergillus brasiliensis* (ATCC 16404), were measured. Polyepsilon-lysine was used as the polylysine, and propylene glycol, butylene glycol, or pentylene glycol was used as the glycol compound.

Specifically, as shown in Table 6 below, preservatives for skin external preparations comprising polyepsilon-lysine, caprylyl glyceryl ether and a glycol compounds at a weight ratio of 0.1 to 5:10 to 30:65 to 75 were prepared. Each of the preservatives for skin external preparations was diluted to about 10%, and each of the dilutions was further serially diluted 1: 1 (12 times). Thereby, samples of various concentrations (about 0.01% to about 10%) for each of the preservatives for skin external preparations were prepared. Each of the obtained samples was added to tryptic soy broth (TSB) medium or potato dextrose agar (PDA) medium. In about 10 ml of the medium to which each sample was added, each type of bacteria or fungi described above was seeded so that the final concentration of the bacteria or fungi was about 10⁶ CFU/ml, and then the bacteria were cultured at about 32°C for about 24 hours to 48 hours, and fungi were cultured at about 25°C for about 24 hours to 48 hours. Thereafter, the minimum inhibitory concentration (MIC) of each sample capable of inhibiting the growth of the bacteria or fungi by about 95% or more was determined by observing the state of the bacterial or fungal culture medium. The growth of normal bacteria in a medium to which no sample was added was measured and used as a positive control.

As a result, as shown in Table 6, it was confirmed that, among the preservatives for skin external preparations comprising polyepsilon-lysine, caprylyl glyceryl ether and the glycol compound at a weight ratio of 0.1 to 5: 10 to 30: 65 to 75, the preservatives comprising propylene glycol, butylene glycol or pentylene glycol as the glycol compound all exhibited excellent antimicrobial activity at remarkably low concentrations. In particular, it was confirmed that the preservatives for skin external preparations comprising polyepsilon-lysine, caprylyl glyceryl ether and propylene glycol at a weight ratio of 0.1 to 1: 25 to 30: 65 to 75 exhibited the best antimicrobial activity against *C. albicans* or *A. brasiliensis.*

**[Table 6]**

| Weight ratio | Example | MIC (%, w/w) of sample | | | | |
|---|---|---|---|---|---|---|
| | | *E. coli* | *P. aeruginosa* | *S. aureus* | *C. albicans* | *A. brasiliensis* |
| Polyepsilon-lysine, caprylyl glyceryl ether, and **propylene glycol** (0.1 to 1: 25 to 30: 65 to 75) | Example 6 or 9 | 0.3 | 3 | 0.2 | 0.1 | 0.1 |
| Polyepsilon-lysine, caprylyl glyceryl ether and **propylene glycol** (3.5 to 5: 20 to 30: 65 to 75) | Example 12 or 15 | 0.4 | 3 | 0.2 | 0.1 | 0.1 |
| Polyepsilon-lysine, caprylyl glyceryl ether and **butylene glycol** (3.5 to 5: 20 to 30: 65 to 75) | Example 27 or 30 | 0.4 | 3 | 0.25 | 0.075 | 0.075 |
| Polyepsilon-lysine, caprylyl glyceryl ether and **pentylene glycol** (3.5 to 5: 20 to 30: 65 to 75) | Example 42 or 45 | 0.4 | 2 | 0.2 | 0.1 | 0.1 |

Through this Experimental Example, it was confirmed that a preservative for a skin external preparation comprising about 0.1 to 5 wt% of polylysine, about 10 to 30 wt% of caprylyl glyceryl ether and about 65 to 75 wt% of the glycol compound exhibited excellent antimicrobial activity at a remarkably low concentration.

In particular, it was confirmed that a preservative for a skin external preparation comprising about 0.1 to 1 wt% of polyepsilon-lysine, about 25 to 30 wt% of caprylyl glyceryl ether and about 65 to 75 wt% of propylene glycol exhibited excellent antimicrobial activity against both bacteria and fungi at a remarkably low concentration.

### 3. Preparation and evaluation of cosmetic compositions containing preservatives for skin external preparations

### Example 51 to Example 56. Preparation of cosmetic compositions containing preservatives for skin external preparations (emulsion and shampoo)

In these Examples, cosmetic compositions containing a preservative for a skin external preparation comprising at least two of polylysine, caprylyl glyceryl ether and a glycol compound, and further containing other cosmetic ingredients, were prepared. Polyepsilon-lysine was used as the polylysine, and propylene glycol was used as the glycol compound.

Specifically, three types of preservatives for skin external preparations comprising at least two of polyepsilon-lysine, caprylyl glyceryl ether, and propylene glycol at varying weight ratios were prepared. Three types of emulsion formulation cosmetic compositions and three types of shampoo formulation cosmetic compositions, which contain about 3 wt% of any one of the three types of preservatives for skin external preparations and the same wt% of other additional cosmetic ingredients, were prepared. The results are shown in Tables 7 and 8 below. In addition, emulsion formulation and shampoo formulation cosmetic compositions containing none of polyepsilon-lysine, caprylyl glyceryl ether and propylene glycol were prepared and used as a control.

**[Table 7]**

| Ingredient name | | Emulsion formulation example (unit: wt%) | | | |
|---|---|---|---|---|---|
| | | **Example 51** | **Example 52** | **Example 53** | Comparative Example 1 |
| Purified water | | Up to 100 | Up to 100 | Up to 100 | Up to 100 |
| Carbomer 940 | | 10 | 10 | 10 | 10 |
| Polyacrylate 13/polyisobutene/polysorbate 20 | | 0.8 | 0.8 | 0.8 | 0.8 |
| PEG-40 hydrogenated castor oil | | 0.8 | 0.8 | 0.8 | 0.8 |
| Phenyl trimethicone | | 1 | 1 | 1 | 1 |
| L-arginine | | 0.16 | 0.16 | 0.16 | 0.16 |
| **Preservative for skin external preparation** | **Example 6 or 9 (polyepsilon-lysine: caprylyl glyceryl ether: propylene glycol = 0.1 to 1: 25 to 30: 65 to 75)** | **3** | - | - | - |
| | **Caprylyl glyceryl ether and propylene glycol (30: 70)** | - | **3** | - | - |
| | **Polyepsilon-lysine and propylene glycol (50: 50)** | - | - | **3** | - |

**[Table 8]**

| Ingredient name | | Shampoo formulation example (unit: wt%) | | | |
|---|---|---|---|---|---|
| | | **Example 54** | **Example 55** | **Example 56** | Comparative Example 2 |
| Purified water | | Up to 100 | Up to 100 | Up to 100 | Up to 100 |
| Polyquatemium-10 | | 0.9 | 0.9 | 0.9 | 0.9 |
| Citric acid | | 0.1 | 0.1 | 0.1 | 0.1 |
| Disodium laureth sulfosuccinate/disodium laureth sulfoacetate | | 7 | 7 | 7 | 7 |
| Decyl poly glucose | | 7 | 7 | 7 | 7 |
| Disodium laureth sulfosuccinate | | 26 | 26 | 26 | 26 |
| Sorbitan stearate | | 1 | 1 | 1 | 1 |
| **Preservative for skin external preparation** | **Example 6 or 9 (polyepsilon-lysine: caprylyl glyceryl ether: propylene glycol = 0.1 to 1: 25 to 30: 65 to 75)** | **3** | - | - | - |
| | **Caprylyl glyceryl ether and propylene glycol (30: 70)** | - | **3** | - | - |
| | **Polyepsilon-lysine and propylene glycol (50: 50)** | - | - | **3** | - |

### Experimental Example 3. Evaluation of preservative activity of cosmetic compositions (emulsion and shampoo) containing preservatives for skin external preparations

In this experimental example, in order to evaluate the preservative activity of the preservatives for a skin external preparation comprising at least two of polylysine, caprylyl glyceryl ether and the glycol compound, a certain number of bacteria were inoculated into the cosmetic compositions of Examples 51 to 56, which each contain the preservative for a skin external preparation, and then the number of remaining viable cells was counted. The cosmetic compositions of Comparative Examples 1 and 2 were used as a control.

Specifically, each of the cosmetic compositions of Examples 51 to 56 and Comparative Examples 1 and 2 was inoculated and mixed with a bacterial culture of *Escherichia coli* (ATCC 8739), *Pseudomonas aeruginosa* (ATCC 9027), or *Staphylococcus aureus* (ATCC 6538) so that the initial concentration of bacteria per sample was about 10⁶ CFU/g. Thereafter, while each sample was cultured in a thermostat at about 30°C to about 32°C for about 4 weeks, about 1 g of each sample was taken at intervals of about 7 days and the number of remaining viable cells was counted.

In addition, each of the cosmetic compositions of Examples 51 to 56 and Comparative Examples 1 and 2 was inoculated and mixed with a fungal culture of the fungus *Aspergillus brasiliensis* (ATCC 16404) so that the initial concentration of fungi per sample was about 10⁵ CFU/g. Thereafter, while each sample was cultured in a thermostat at about 25°C for about 4 weeks, the presence or absence of off-odor and the occurrence of hyphae and spores on the sample surface were checked for each sample at intervals of about 7 days.

As a result, as shown in Table 9 below, it was confirmed that the cosmetic compositions of Examples 51 and 54, which contain the preservative for a skin external preparation comprising polyepsilon-lysine, caprylyl glyceryl ether and propylene glycol at a weight ratio of 0.1 to 1: 25 to 30: 65 to 75, exhibited significantly better preservative activity against bacteria and fungi than the cosmetic compositions of Examples 52, 53, 55 and 56 and Comparative Examples 1 and 2, which contain the preservative for a skin external preparation comprising only two selected from among polyepsilon-lysine, caprylyl glyceryl ether and propylene glycol or contain none of polyepsilon-lysine, caprylyl glyceryl ether and propylene glycol.

**[Table 9]**

| Cosmetic composition | | Bacteria (cfu/g) | | | | | Fungi |
|---|---|---|---|---|---|---|---|
| | | Day 0 | Day 7 | Day 14 | Day 21 | Day 28 | |
| Emulsion formulation | Example 51 | 2.7 x 10⁶ | 0 | 0 | 0 | 0 | - |
| | Example 52 | 2.7 x 10⁶ | <10 | 0 | 0 | 0 | ++ |
| | Example 53 | 2.7 x 10⁶ | <10 | 0 | 0 | 0 | ++ |
| | Comparative Example 1 | 2.7 x 10⁶ | 2.1 x 10⁶ | 3.3 x 10⁶ | 3.8 x 10⁶ | 1.1 x 10⁶ | +++ |
| Shampoo formulation | Example 54 | 2.7 x 10⁶ | 0 | 0 | 0 | 0 | - |
| | Example 55 | 2.7 x 10⁶ | <10 | 0 | 0 | 0 | ++ |
| | Example 56 | 2.7 x 10⁶ | <10 | 0 | 0 | 0 | ++ |
| | Comparative Example 2 | 2.7 x 10⁶ | 1.9 x 10⁶ | 4.4 x 10⁵ | 2.3 x 10⁶ | 6.1 x 10⁵ | +++ |

| | | | | | | | |
|---|---|---|---|---|---|---|---|
| (-: steady state with no off-odor, hyphae, and spores for 8 weeks; +: occurrence of mold on the walls or lid within 4 weeks; ++: occurrence of off-odor and occurrence of mold on a part of the sample surface within 4 weeks; +++: occurrence of off-odor and occurrence of mold on the entire surface of the sample within 4 weeks) | | | | | | | |

Through this Experimental Example, it was confirmed that, when the preservative for a skin external preparation comprising about 0.1 to 1 wt% of polylysine, about 25 to 30 wt% of caprylyl glyceryl ether and about 65 to 75 wt% of the glycol compound was included to the emulsion and shampoo formulation cosmetic compositions, it exhibited significantly good preservative activity against bacteria and fungi, and that, when the wt% of each of polylysine, caprylyl glyceryl ether and the glycol compound in the preservative for a skin external preparation was out of the above wt% range, the preservative in the emulsion and shampoo formulation cosmetic compositions exhibited significantly reduced preservative activity against bacteria and fungi.

### Example 57 to Example 60. Preparation of cosmetic compositions containing preservatives for skin external preparations (cream)

In these Examples, cosmetic compositions containing a preservative for a skin external preparation comprising polylysine, caprylyl glyceryl ether and a glycol compound, and further containing other cosmetic ingredients, were prepared. Polyepsilon-lysine was used as the polylysine, and propylene glycol was used as the glycol compound.

Specifically, four types of preservatives for skin external preparations comprising polyepsilon-lysine, caprylyl glyceryl ether and propylene glycol at varying weight ratios were prepared. Four types of cream formulation cosmetic compositions, which contain about 3 wt% of any one of the four types of preservatives for skin external preparations and the same wt% of other additional cosmetic ingredients, were prepared. The results are shown in Table 10 below. In addition, a cream formulation cosmetic composition containing none of polyepsilon-lysine, caprylyl glyceryl ether and propylene glycol was prepared and used as a control.

**[Table 10]**

| Ingredient name | | Cream formulation example (unit: wt%) | | | | |
|---|---|---|---|---|---|---|
| | | Example | Example | Example | Example | Comparative |
| | | 57 | 58 | 59 | 60 | Example 3 |
| Purified water | | Up to 100 | Up to 100 | Up to 100 | Up to 100 | Up to 100 |
| Disodium EDTA | | 0.02 | 0.02 | 0.02 | 0.02 | 0.02 |
| Glycerin | | 5 | 5 | 5 | 5 | 5 |
| Glyceryl stearate/PEG-100 stearate | | 1 | 1 | 1 | 1 | 1 |
| Caprylic/capric triglyceride | | 5 | 5 | 5 | 5 | 5 |
| Sodium hyaluronate | | 0.1 | 0.1 | 0.1 | 0.1 | 0.1 |
| Polyacrylate 13/polyisobutene/polysorbate 20 | | 1 | 1 | 1 | 1 | 1 |
| Polysorbate 60 | | 2 | 2 | 2 | 2 | 2 |
| Squalane | | 3 | 3 | 3 | 3 | 3 |
| Phenyl trimethicone | | 0.5 | 0.5 | 0.5 | 0.5 | 0.5 |
| **Preservative for skin external preparation (polyepsilon-lysine: caprylyl glyceryl ether: propylene glycol)** | **Example 6 or 9 (0.1 to 1: 25 to 30: 65 to 75)** | **3** | - | - | - | - |
| | **8: 10: 82** | - | **3** | - | - | - |
| | **4: 20: 76** | - | - | **3** | - | - |
| | **Example 11, 13 or 16 (1 to 5: 35 to 45: 53 to 58)** | - | - | - | **3** | - |

### Experimental Example 4. Evaluation of preservative activity of cosmetic compositions (cream) containing preservatives for skin external preparations

In this experimental example, in order to evaluate the preservative activity of the preservative for a skin external preparation comprising polylysine, caprylyl glyceryl ether and the glycol compound, a certain number of bacteria were inoculated into the cosmetic compositions of Examples 57 to 60, which each contain the preservative for a skin external preparation, and then the number of remaining viable cells was counted. The cosmetic composition of Comparative Example 3 was used as a control.

Specifically, each of the cosmetic compositions of Examples 57 to 60 and Comparative Example 3 was inoculated and mixed with a bacterial culture of *Escherichia coli* (ATCC 8739), *Pseudomonas aeruginosa* (ATCC 9027), or *Staphylococcus aureus* (ATCC 6538) so that the initial concentration of bacteria per sample was about 10⁶ CFU/g. Thereafter, while each sample was cultured in an thermostat at about 30°C to about 32°C for about 4 weeks, about 1 g of each sample was taken at intervals of about 7 days and the number of remaining viable cells was counted.

In addition, each of the cosmetic compositions of Examples 57 to 60 and Comparative Example 3 was inoculated and mixed with a fungal culture of the fungus *Aspergillus brasiliensis* (ATCC 16404) so that the initial concentration of fungi per sample was about 10⁵ CFU/g. Thereafter, while each sample was cultured in **a** thermostat at about 25°C for about 4 weeks, the presence or absence of off-odor and the occurrence of hyphae and spores on the sample surface were checked for each sample at intervals of about 7 days.

As a result, as shown in Table 11 below, it was confirmed that the cosmetic composition of Example 57, which contains the preservative for a skin external preparation comprising polyepsilon-lysine, caprylyl glyceryl ether and propylene glycol at a weight ratio of 0.1 to 1: 25 to 30: 65 to 75, exhibited significantly good preservative activity against bacteria and fungi. On the other hand, it was confirmed that the cosmetic compositions of Examples 58, 59 and 60 and Comparative Example 3, which each contain a preservative for a skin external preparation comprising polyepsilon-lysine, caprylyl glyceryl ether and propylene glycol at a weight ratio out of the above range of 0.1 to 1: 25 to 30: 65 to 75 or comprising none of polyepsilon-lysine, caprylyl glyceryl ether and propylene glycol, exhibited significantly reduced preservative activity against bacteria and fungi.

**[Table 11]**

| Cosmetic composition | | Bacteria (cfu/g) | | | | | Fungi |
|---|---|---|---|---|---|---|---|
| | | Day 0 | Day 7 | Day 14 | Day 21 | Day 28 | |
| Cream formulation | **Example 57** | 2.4 x 10⁶ | 0 | 0 | 0 | 0 | - |
| | **Example 58** | 2.5 x 10⁶ | 1.5 x 10⁶ | 2.1 x 10⁵ | 2.8 x 10⁵ | 3.1 x 10⁵ | +++ |
| | **Example 59** | 2.7 x 10⁶ | 1.9 x 10⁶ | 1.1 x 10⁶ | 2.5 x 10⁵ | 2.1 x 10⁶ | +++ |
| | **Example 60** | 2.5 x 10⁶ | 1.6 x 10⁵ | 5.6 x 10² | 0 | 0 | ++ |
| | Comparative Example 3 | 2.7 x 10⁶ | 2.4 x 10⁶ | 3.1 x 10⁶ | 5.1 x 10⁶ | 2.8 x 10⁶ | +++ |

| | | | | | | | |
|---|---|---|---|---|---|---|---|
| (-: steady state with no off-odor, hyphae, and spores for 8 weeks; +: occurrence of mold on the walls or lid within 4 weeks; ++: occurrence of off-odor and occurrence of mold on a part of the sample surface within 4 weeks; +++: occurrence of off-odor and occurrence of mold on the entire surface of the sample within 4 weeks) | | | | | | | |

Through this Experimental Example, it was confirmed that, when the preservative for a skin external preparation comprising about 0.1 to 1 wt% of polylysine, about 25 to 30 wt% of caprylyl glyceryl ether and about 65 to 75 wt% of the glycol compound was added to the cream formulation cosmetic composition, it exhibited significantly good preservative activity against bacteria and fungi, and that, when the wt% of each of polylysine, caprylyl glyceryl ether and the glycol compound in the preservative for a skin external preparation was out of the above wt% range, the preservative in the cream formulation cosmetic composition exhibited significantly reduced preservative activity against bacteria and fungi.

Through the above Examples and Experimental Examples, it was confirmed that the preservative for a skin external preparation comprising polylysine, caprylyl glyceryl ether, a glycol compound, or a combination thereof could exhibit excellent antimicrobial and preservative effects, and was also safe for the skin because it had a very low skin irritation potential, suggesting that it is suitable for use in a cosmetic composition.

In particular, it was confirmed that the preservative for a skin external preparation comprising polylysine, caprylyl glyceryl ether and a glycol compounds at a weight ratio of 0.1 to 5: 10 to 30: 65 to 75, specifically 0.1 to 1: 25 to 30: 65 to 75, exhibited remarkably good antimicrobial and preservative effects against not only bacteria but also fungi, and at the same time, exhibited optimized formulation stability, suggesting that it can be stably applied to cosmetic compositions as a preservative, an antiseptic or an antiseptic adjuvant.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A preservative for a skin external preparation comprising polylysine and caprylyl glyceryl ether.

2. The preservative according to claim 1, wherein a weight ratio between the polylysine and the caprylyl glyceryl ether is 0.1 to 5: 10 to 30.

3. The preservative according to claim 1, wherein the preservative further comprises a glycol compound.

4. The preservative according to claim 3, wherein a weight ratio between the polylysine, the caprylyl glyceryl ether and the glycol compound is 0.1 to 5: 10 to 30: 65 to 75.

5. The preservative according to claim 3, wherein the glycol compound is at least one selected from the group consisting of ethylene glycol, diethylene glycol, propylene glycol, dipropylene glycol, butylene glycol, pentylene glycol, hexylene glycol, ethoxydiglycol, and an alkanediol having 3 to 12 carbon atoms.

6. The preservative according to claim 3, wherein the glycol compound is at least one selected from the group consisting of propylene glycol, butylene glycol, pentylene glycol, propanediol, butanediol, and pentanediol.

7. A cosmetic composition containing the preservative for a skin external preparation according to any one of claims 1 to 6.

8. The cosmetic composition according to claim 7, wherein the cosmetic composition contains the preservative for a skin external preparation in an amount of 0.0001 wt% to 50 wt% based on the total weight of the cosmetic composition.

9. The cosmetic composition according to claim 7, wherein the formulation of the cosmetic composition is a solution, emulsion, suspension, softening lotion, nourishing lotion, massage cream, nourishing cream, pack, gel, skin-adhesive type cosmetic, lipstick, powder, makeup base, foundation, shampoo, rinse, scalp cleanser, scalp lotion, scalp cream, scalp pack, scalp gel, scalp ointment, scalp gel, body cleanser, soap, toothpaste, mouth freshener, paste, lotion, ointment, gel, cream, patch, spray, or aerosol formulation.

10. The cosmetic composition according to claim 7, wherein the cosmetic composition further contains an additive selected from the group consisting of purified water, a carrier, an emulsifier, a moisturizer, a skin conditioning agent, a surfactant, a chelating agent, an antioxidant, an antiseptic, a bactericide, a stabilizer, a preservative, a pH adjusting agent, a lubricant, a solubilizer, a solvent, and combinations thereof.
